# EUROPEAN PATENT APPLICATION

(11) **EP 3 974 438 A1**
(43) Date of publication of application: **30.03.2022**
(21) Application number: 20810508.0
(22) Date of filing: 19.05.2020
(51) Int. Cl.: C07K 5/06, C07K 5/062, C07K 5/078, G01N 21/64, G01N 21/78, C12Q 1/37, C09B 11/28, G01N 33/48

(54) **FLUORESCENT PROBE FOR USE IN DETECTION OF BRAIN TUMOR**

(30) Priority: 21.05.2019 JP 2019095102
(71) Applicant: The University of Tokyo, Bunkyo-ku, Tokyo 113-8654 (JP)
(72) Inventor: KITAGAWA Yosuke, Tokyo 113-8654 (JP); TANAKA Shota, Tokyo 113-8654 (JP); SAITO Nobuhito, Tokyo 113-8654 (JP); KURIKI Yugo, Tokyo 113-8654 (JP); KAMIYA Mako, Tokyo 113-8654 (JP); URANO Yasuteru, Tokyo 113-8654 (JP)
(74) Representative: Symbiosis IP Limited
(86) International application number: PCT/JP2020/019814
(87) International publication number: WO 2020/235567

(57) **Abstract**

Provided is a novel fluorescent probe which can be used in a spray manner, has an outstandingly high sensitivity- specificity with instantaneousness, and enables detection of a brain tumor.

A fluorescent probe for detecting a brain tumor, including a compound of the following formula (I) or a salt thereof: 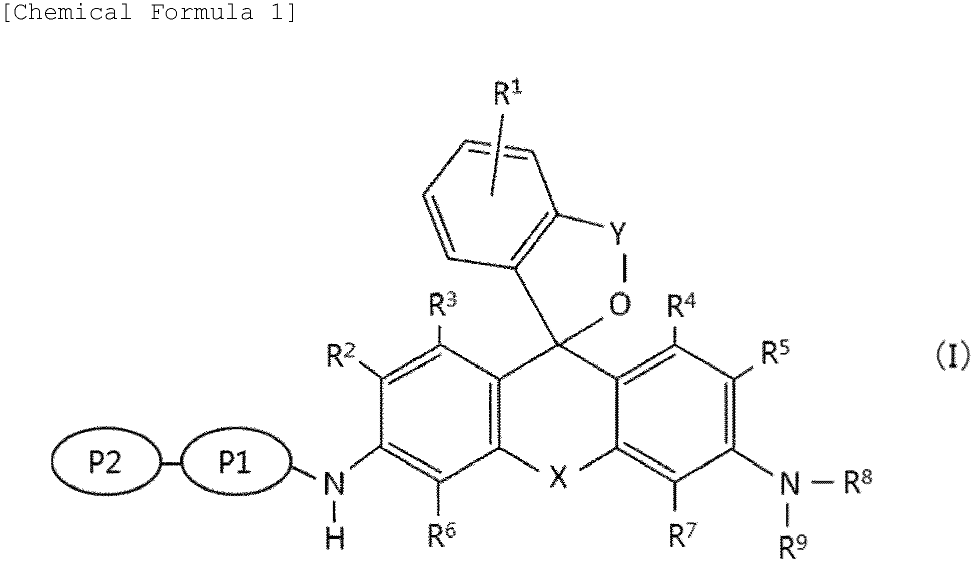 wherein P1 represents an arginine residue, a histidine residue or a tyrosine residue, P2 represents a proline residue or a glycine residue, where P1 is linked to an adjacent N atom by forming an amide bond, and P2 is linked to P1 by forming an amide bond; R¹ represents a hydrogen atom, or 1 to 4 identical or different substituents each independently selected from the group consisting of an optionally substituted alkyl group, a carboxyl group, an ester group, an alkoxy group, an amide group and an azide group; R², R³, R⁴, R⁵, R⁶ and R⁷ each independently represent a hydrogen atom, a hydroxyl group, an optionally substituted alkyl group or a halogen atom; R⁸ and R⁹ each independently represent a hydrogen atom or an alkyl group;
X represents O, Si(R^{a})(R^{b}), Ge(R^{a})(R^{b}), Sn(R^{a})(R^{b}), C(R^{a})(R^{b})or P(=O)(R^{a}); R^{a} and R^{b} each independently represent a hydrogen atom, an alkyl group or an aryl group; and Y represents a C₁-C₃ alkylene group.

## Description

### Technical Field

The present invention relates to a fluorescent probe for detecting a brain tumor. More specifically, the present invention relates to a fluorescent probe capable of specifically detecting and labeling a brain tumor by applying the fluorescent probe to a tissue specimen, and a detection method using the fluorescent probe.

### Background Art

Glioma account for about 30% of primary brain tumors, and the median survival duration in glioblastoma, the most malignant type of glioma, is about 1.5 years. Even in low-grade glioma, complete cure is rarely achieved, and glioma often becomes more malignant in several years and fatal in 5 to 10 years. In glioma surgery, complete removal is a good prognostic factor, but excision cannot be extensively extended to the surrounding brain because of functional localization. It is often difficult to achieve both improvement of the removal ratio and preservation of the brain function, which are in a trade-off relationship.

Visualization of the tumor is attempted to enable safe maximum removal. So far, as a fluorescent reagent for visualizing glioma, only 5-aminolevulinic acid (5-ALA) has been covered by insurance. However, methods using 5-ALA have issues such as sensitivity-specificity limitations, necessity of preoperative internal administration, a problem about fluorescence duration associated with metabolism, difficulty of re-administration, and inability to perform fluorescent labeling in the case of low-grade tumors (e.g. Non-Patent Literatures 1 and 2). In addition, in conventional methods of tumor visualization, it is difficult to achieve both improvement of the removal ratio and preservation of the cranial nerve function, which are in a trade-off relationship.

### Citation List

### Non Patent Literature

Non Patent Literature 1: Coburger et al., Neurosurgical focus, 36.2 (2014): E3
Non-Patent Document 2: Ferraro et al., Neurosurgical review, 39.4 (2016): 545-555

### Disclosure of Invention

### Technical Problem

Thus, it is desired to develop a fluorescent probe which can be used in place of conventional 5-ALA, enables brain tumors to be diagnosed and visualized by application or topical spraying, and has high safety. Accordingly, an object of the present invention is to provide a novel fluorescent probe which can be used in a topical manner, has a high sensitivity-specificity with instantaneousness, and enables detection of a brain tumor.

### Solution to Problem

The present inventors have extensively conducted studies for solving the aforementioned problems, and resultantly found that a fluorescent probe, having a structure in which a specific amino acid residue is introduced as a side chain into a xanthene-like skeleton, exhibits a specific fluorescent response to tumor cells or tissues of a brain tumor, and is useful for labeling and diagnosing it. In addition, the present inventors have found that by using the fluorescent probe, various problems of the conventional fluorescent labeling method can be solved, and the novel technique for supporting brain tumor surgery can complement the conventional fluorescent labeling method. On the basis of these findings, the present invention has been completed.

That is, in one aspect, the present invention provides:
<1> a fluorescent probe for detecting a brain tumor, including a compound of the following formula (I) or a salt thereof: 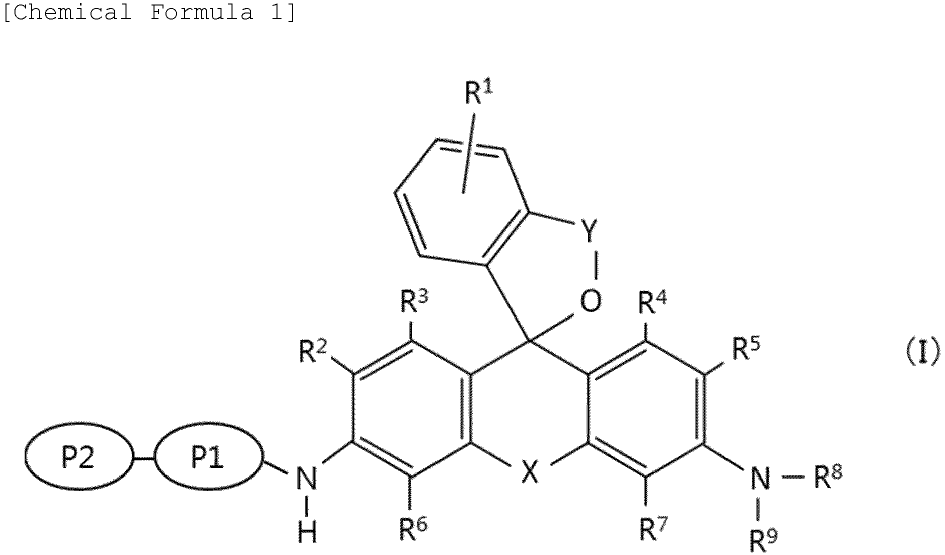 wherein P1 represents an arginine residue, a histidine residue or a tyrosine residue, P2 represents a proline residue or a glycine residue, where P1 is linked to an adjacent N atom by forming an amide bond, and P2 is linked to P1 by forming an amide bond; R¹ represents a hydrogen atom, or 1 to 4 identical or different substituents each independently selected from the group consisting of an optionally substituted alkyl group, a carboxyl group, an ester group, an alkoxy group, an amide group and an azide group; R², R³, R⁴, R⁵, R⁶ and R⁷ each independently represent a hydrogen atom, a hydroxyl group, an optionally substituted alkyl group or a halogen atom; R⁸ and R⁹ each independently represent a hydrogen atom or an alkyl group; X represents O, Si(R^{a})(R^{b}), Ge(R^{a})(R^{b}), Sn(R^{a})(R^{b}), C(R^{a})(R^{b})or P(=O)(R^{a}); R^{a} and R^{b} each independently represent a hydrogen atom, an alkyl group or an aryl group; and Y represents a C₁-C₃ alkylene group;
<2> the fluorescent probe according to <1>, in which the brain tumor is glioblastoma or glioma;
<3> the fluorescent probe according to <1> or <2>, for identifying a brain tumor or glioma by detecting the presence of a protease expressed in tumor cells;
<4> the fluorescent probe according to <3>, in which the protease is calpain 1 or cathepsin D;
<5> the fluorescent probe according to any one of <1> to <4>, in which X is O (oxygen atom) and Y is a methylene group;
<6> the fluorescent probe according to any one of <1> to <5>, in which all of R¹, R², R³, R⁴, R⁵, R⁶, R⁷, R⁸ and R⁹ are hydrogen atoms; and
<7> an application-type or topical spray-type composition for detection of a tumor, including a solution of the fluorescent probe according to any one of <1> to <6>.

In another aspect, the present invention also relates to a method for detecting or visualizing a brain tumor or glioma, and provides:
<8> a method for detecting or visualizing a brain tumor, including the steps of: bringing the fluorescent probe according to any one of <1> to <6> into contact with a target tissue; and observing a fluorescent response caused by a reaction between a protease expressed in the target tissue and the fluorescent probe;
<9> the method according to <8>, in which the step of bringing the fluorescent probe into contact with the target tissue is carried out by topically applying or spraying a solution containing the fluorescent probe to the target tissue;
<10> the method according to <8> or <9>, including visualizing the fluorescent response using fluorescence imaging means;
<11> the method according to any one of <8> to <10>, in which the brain tumor is glioblastoma or glioma; and
<12> the method according to any one of <8> to <11>, in which the protease expressed in the target tissue is calpain 1 or cathepsin D.

### Advantageous Effects of Invention

According to the fluorescent probe of the present invention, a brain tumor can be specifically and sensitively detected and labeled semi-instantaneously. The fluorescent probe of the present invention can be directly and topically applied or sprayed to a brain tumor, and therefore has an exceptional effect of acknowledging the presence of a brain tumor or increases the possibility to be identified and imaged accurately, quickly and with high sensitivity by a simple method regardless of the dosage form.

Thus, improvement of the removal ratio and improvement of the overall survival rate by labeling an affected part such as a brain tumor during surgery can be expected. In addition, it is also possible to solve the problem that currently, there is not a fluorescent probe that can be labeled for low-grade glioma.

In addition, a detection method using the fluorescent probe of the present invention is superb in usefulness in that the detection procedure is simple, the detection can be performed by visible light which is safe for living bodies, and the amount of the fluorescent probe to be used is very small.

### Brief Description of Drawings

Fig. 1 is a fluorescent image obtained when a group of fluorescent probe compounds is added to a fresh tumor tissue and a peripheral tissue.
Fig. 2 is a graph obtained by plotting a change in fluorescence intensity obtained when a group of fluorescent probe compounds is added to a fresh tumor tissue and a peripheral tissue.
Fig. 3 is a diagram showing the presence or absence of a fluorescent response for a group of fluorescent probe compounds.
Fig. 4 is a graph showing a change in fluorescence intensity by a reaction with a fluorescent probe YK190 for calpain 1 and cathepsin D.
Fig. 5 is a fluorescent image when transfection of SiRNA with lipofectamine is performed using a U 87 glioblastoma cell line, and reaction with a fluorescent probe is observed after culture.
Fig. 6 is a graph showing an enzyme expression level in real-time PCR.

### Description of Embodiments

Hereinafter, embodiments of the present invention will be described. The scope of the present invention is not limited by the description, and besides the examples shown below, changes can be appropriately made as long as the spirit of the present invention is not impaired.

### 1. Definitions

Herein, the "amino acid residue" is a structure corresponding to a partial structure (acyl group) remaining after removal of a hydroxyl group from a carboxyl group of an amino acid. In addition, as for the "amino acid", any compound can be used as long as it has both an amino group and a carboxyl group, and the amino acid includes natural and non-natural compounds. The amino acid may be any of a neutral amino acid, a basic amino acid and an acidic amino acid, and it is possible to use amino acids which themselves function as a transmitter such as a neurotransmitter, and amino acids which are constituents of polypeptide compounds such as physiologically active peptides (including dipeptides, tripeptides, tetrapeptides and oligopeptides) or proteins. The amino acid may be, for example, an α amino acid, a β amino acid, a γ amino acid or the like. It is preferable to use an optically active amino acid as the amino acid. For example, as the α amino acid, either a D- or a L-amino acid may be used, and it may be preferable to select an optically active amino acid that functions in a living body.

Herein, the "halogen atom" means a fluorine atom, a chlorine atom, a bromine atom or an iodine atom.

Herein, the "alkyl group" may be any of linear, branched and cyclic aliphatic hydrocarbon groups or combinations thereof. The number of carbon atoms in the alkyl group is not particularly limited, and is, for example, 1 to 20 (C₁₋₂₀), 3 to 15 (C₃₋₁₅) or 5 to 10 (C₅₋₁₀). A specified number of carbon atoms means an "alkyl" having the specified number of carbon atoms. For example, C₁₋₈ alkyls include methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl and the like. Herein, the alkyl group may have one or more arbitrary substituents. Examples of the substituent include, but are not limited to, alkoxy groups, halogen atoms, amino groups, mono- or di-substituted amino groups, substituted silyl groups and acyl. When the alkyl group has two or more substituents, the substituents may be the same or different. The same applies to alkyl moieties of other substituents including alkyl moieties (e.g. alkoxy groups and arylalkyl groups).

Herein, when a certain functional group is defined as "optionally having a substituent", the type of substituent, the substitution position and the number of substituents are not particularly limited, and two or more substituents are present, they may be the same or different. Examples of the substituent include but are not limited to, alkyl groups, alkoxy groups, hydroxyl groups, carboxyl groups, halogen atoms, sulfo groups, amino groups, alkoxycarbonyl groups and oxo groups. These substituents may further have substituents. Examples of such groups include, but are not limited to, halogenated alkyl groups and dialkylamino groups.

Herein, the "alkylene" is a divalent group composed of a linear or branched saturated hydrocarbon, and examples thereof include methylene, 1-methylmethylene, 1,1-dimethylmethylene, ethylene, 1-methylethylene, 1-ethylethylene, 1,1-dimethylethylene, 1,2-dimethylethylene, 1,1-diethylethylene, 1,2-diethylethylene, 1-ethyl-2-methylethylene, trimethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, tetramethylene, 1-methyltetramethylene, 2-methyltetramethylene, 1,1-dimethyltetramethylene, 1,2-dimethyltetramethylene, 2,2-dimethyltetramethylene and 2,2-di-n-propyltrimethylene.

Herein, the "alkoxy group" is a structure in which the alkyl group is bonded to an oxygen atom, and examples thereof include saturated linear, branched and cyclic alkoxy groups or combinations thereof. Examples of suitable groups include a methoxy group, an ethoxy group, a n-propoxy group, an isopropoxy group, a cyclopropoxy group, a n-butoxy group, an isobutoxy group, a s-butoxy group, a t-butoxy group, a cyclobutoxy group, a cyclopropylmethoxy group, a n-pentyloxy group, a cyclopentyloxy group, a cyclopropylethyloxy group, a cyclobutylmethyloxy group, a n-hexyloxy group, a cyclohexyloxy group, a cyclopropylpropyloxy group, a cyclobutylethyloxy group and a cyclopentylmethyloxy group.

Herein, the "aryl" may be either a monocyclic aromatic hydrocarbon group or a condensed polycyclic aromatic hydrocarbon group, and may be an aromatic heterocyclic ring containing one or more heteroatoms (e.g. an oxygen atom, a nitrogen atom or a sulfur atom) as ring constituent atoms. Here, the aromatic heterocyclic ring may also be referred to as "heteroaryl" or "heteroaromatic". The aryl may be attached at all possible positions, whether it is a monocyclic ring or a condensed ring. Non-limiting examples of the monocyclic aryl include a phenyl group (Ph), a thienyl group (2- or 3-thienyl group), a pyridyl group, a furyl group, a thiazolyl group, an oxazolyl group, a pyrazolyl group, a 2-pyrazinyl group, a pyrimidinyl group, a pyrrolyl group, an imidazolyl group, a pyridazinyl group, a 3-isothiazolyl group, a 3-isoxazolyl group, a 1,2,4-oxadiazol-5-yl group and a 1,2,4-oxadiazol-3-yl group. Non-limiting examples of the condensed polycyclic aryl include a 1-naphthyl group, a 2-naphthyl group, a 1-indenyl group, a 2-indenyl group, a 2,3-dihydroindene-1-yl group, a 2,3-dihydroindene-2-yl group, a 2-anthryl group, an indazolyl group, a quinolyl group, an isoquinolyl group, a 1,2-dihydroisoquinolyl group, a 1,2,3,4-tetrahydroisoquinolyl group, an indolyl group, an isoindolyl group, a phthalazinyl group, a quinoxalinyl group, a benzofuranyl group, a 2,3-dihydrobenzofuran-1-yl group, a 2,3-dihydrobenzofuran-2-yl group, a 2,3-dihydrobenzothiophene-1-yl group, a 2,3-dihydrobenzothiophene-2-yl group, a benzothiazolyl group, a benzimidazolyl group, a fluorenyl group and a thioxanthenyl group. Herein, the aryl group may have one or more arbitrary substituents on the ring thereof. Examples of the substituent include, but are not limited to, alkoxy groups, halogen atoms, amino groups, mono- or di-substituted amino groups, substituted silyl groups and acyl. When the aryl group has two or more substituents, the substituents may be the same or different. The same applies to aryl moieties of other substituents including aryl moieties (e.g. aryloxy groups and arylalkyl groups).

Herein, "alkylamino" and "arylamino" mean an amino group in which a hydrogen atom of a -NH₂ group is substituted with one or two of the aforementioned alkyls or aryls. Examples thereof include methylamino, dimethylamino, ethylamino, diethylamino, ethylmethylamino, and benzylamino. Similarly, "alkylthio" and "arylthio" mean a group in which a hydrogen atom of a -SH group is substituted with any of the aforementioned alkyls or aryls. Examples thereof include methylthio, ethylthio and benzylthio.

As used herein, the "amide" includes both RNR'CO-(alkylaminocarbonyl- when R is an alkyl) and RCONR'-(alkylcarbonylamino- when R is an alkyl).

As used herein, the "ester" includes both ROCO-(alkoxycarbonyl- when R is an alkyl) and RCOO-(alkylcarbonyloxy- when R is an alkyl).

Herein, the term "ring structure" means a heterocyclic or carbocyclic group when formed by a combination of two substituents, and such a group can be saturated, unsaturated or aromatic. Therefore, the ring structure includes cycloalkyls, cycloalkenyls, aryls and heteroaryls as defined previously. Examples thereof include cycloalkyls, phenyls, naphthyls, morpholinyls, piperdinyls, imidazolyls, pyrrolidinyls and pyridyls. Herein, a substituent can form a ring structure with another substituent, and when such substituents are bonded to each other, a person skilled in the art can understand that a specific substitution, for example, a bond to hydrogen is formed. Therefore, when it is described that specific substituents together form a ring structure, a person skilled in the art can understand that the ring structure can be formed by a usual chemical reaction and is easily generated. Such ring structures and their formation processes are all within the knowledge of a person skilled in the art.

### 2. Fluorescent probe molecule

The fluorescent probe for detecting a brain tumor according to the present invention is characterized by having a structure in which a dipeptide site as a substrate for a specific protease is introduced as a side chain into a xanthene-like skeleton, and in one aspect, the fluorescent probe contains a compound having a structure of the following formula (I) or a salt thereof.

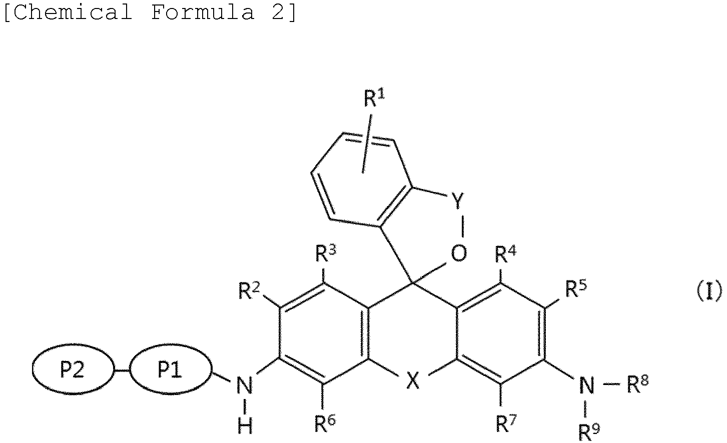

In the general formula (I), P1 represents an arginine residue, a histidine residue, or a tyrosine residue, and P2 represents a proline residue or a glycine residue. The dipeptide site composed of P1 and P2 is selected in the context of a combination of amino acid residues which are likely to be easily hydrolyzed by a certain protease (peptidase) that is specifically expressed in glioblastoma, a type of brain tumor, as demonstrated in Examples described later. The protease that cleaves the dipeptide site composed of P1 and P2 is preferably calpain 1 or cathepsin D. Therefore, in the combination of P1 and P2, it is preferable that P1 is an arginine residue, a histidine residue or a tyrosine residue and P2 is a proline residue or a glycine residue, and it is more preferable that P1 is an arginine residue and P2 is a proline residue.

Here, P1 is linked to adjacent NH in the formula by forming an amide bond, i.e. a carbonyl moiety of the amino acid residue P1 and NH in the formula (I) are linked to a xanthene skeleton by forming an amide bond. Further, P1 can be linked to P2 similarly to a normal peptide chain, and as a result, P2 is linked to P1 by forming an amide bond. As described previously, the "amino acid residue" is a structure corresponding to a partial structure remaining after removal of a hydroxyl group from a carboxyl group of an amino acid. Therefore, P2 has the same structure as that of a so-called N-terminal residue, and P1 which is an intermediate amino acid residue can be linked to P2 similarly to a normal peptide chain.

In the general formula (I), R¹ represents a hydrogen atom, or 1 to 4 identical or different substituents each independently selected from the group consisting of an optionally substituted alkyl group, a carboxyl group, an ester group, an alkoxy group, an amide group and an azide group. When having two or more substituents on a benzene ring, the substituents may be the same or different. R¹ is preferably a hydrogen atom.

R², R³, R⁴, R⁵, R⁶ and R⁷ each independently represent a hydrogen atom, a hydroxyl group, an alkyl group or a halogen atom. R³ and R⁴ are preferably hydrogen atoms. In addition, R², R⁵, R⁶ and R⁷ are preferably hydrogen atoms. More preferably, all of R², R³, R⁴, R⁵, R⁶ and R⁷ are hydrogen atoms.

R⁸ and R⁹ each independently represent a hydrogen atom or an alkyl group. When both R⁸ and R⁹ each represent an alkyl group, they may be the same or different. For example, it is preferable that both R⁸ and R⁹ are hydrogen atoms, and it is preferable that R⁸ is an alkyl group and R⁹ is a hydrogen atom. More preferably, both R⁸ and R⁹ are hydrogen atoms.

In the formula (I), X represents O, Si(R^{a})(R^{b}), Ge(R^{a})(R^{b}), Sn(R^{a})(R^{b}), C(R^{a})(R^{b})or P(=O)(R^{a}). Here, R^{a} and R^{b} each independently represent a hydrogen atom, an alkyl group or an aryl group. When R^{a} and R^{b} are alkyl groups, they may have one or more substituents, and may have, for example, one or more alkyl groups, alkoxy groups, halogen atoms, hydroxyl groups, carboxyl groups, amino groups, sulfo groups and the like as such substituents. R^{a} and R^{b} may each be an optionally substituted alkyl group having 1 to 6 carbon atoms, and each is preferably a methyl group. In some cases, R^{a} and R^{b} may be bonded to each other to form a ring structure. For example, when both R^{a} and R^{b} are alkyl groups, R^{a} and R^{b} can be bonded to each other to form a spiro carbocycle. The ring to be formed is preferably, for example, about a 5- to 8-membered ring. X is preferably O (oxygen atom).

Y represents a C₁-C₃ alkylene group. The alkylene group may be either a linear alkylene group or a branched alkylene group. For example, a methylene group (-CH₂-), an ethylene group (-CH₂-CH₂-), a propylene group (-CH₂-CH₂-CH₂-), and branched alkylene groups such as -CH(CH₃)-, -CH₂-CH(CH₃)- and -CH(CH₂CH₃)- can be used. Of these, a methylene group or an ethylene group is preferable, and a methylene group is more preferable.

The compound represented by the general formula (I) may be present as a salt. Examples of the salt include base addition salts, acid addition salts, and amino acid salts. Examples of the base addition salt include metal salts such as sodium salts, potassium salts, calcium salts and magnesium salts, ammonium salts, and organic amine salts such as triethylamine salts, piperidine salts and morpholine salts, and examples of the acid addition salt include mineral acid salts such as hydrochlorides, sulfates and nitrates, and organic acid salts such as carboxylates, methanesulfonates, paratoluene sulfonates, citrates and oxalates. Examples of the amino acid salt include glycine salts. However, the compound as a salt is not limited to these salts.

The compound of the formula (I) may have one or more asymmetric carbons depending on the type of substituent, and a stereoisomer such as an optical isomer or a diastereoisomer may be present. Stereoisomers in pure form, any mixture of stereoisomers, racemates and the like are all within the scope of the present invention.

The compound of the formula (I) or a salt thereof may be present as a hydrate or a solvate, and any of these substances is within the scope of the present invention. The type of solvent forming the solvate is not particularly limited, and examples thereof include solvents such as ethanol, acetone and isopropanol.

The compound of the general formula (I) can be easily produced by using, for example, a xanthene compound having amino groups at the 3-position and the 6-position and having a 2-carboxyphenyl group or a 2-alkoxycarbonylphenyl group at the 9-position as a raw material, converting the 2-carboxyphenyl group or the 2-alkoxycarbonylphenyl group at the 9-position into a hydroxyalkyl group, and then acylating the amino group at the 3-position. Examples of the 3,6-diaminoxanthene compound that can be used as a raw material include, but are not limited to, rhodamine 110 and rhodamine 123 both of which are commercially available, and an appropriate xanthene compound can be selected according to the structure of a target compound. In addition, a fluorescent probe having the same function as that of the compound of the general formula (I) in the present invention can be produced using a compound having a skeleton in which an oxygen atom of a xanthene skeleton moiety in the compound of the general formula (I) is substituted with a C atom or a Si atom having a specific substituent, or a Ge atom or a Pb atom.

In addition, examples herein specifically show a method for production of a representative compound included in the inventive compounds of the general formula (I), and therefore a person skilled in the art can easily produce any compound included in the general formula (I) by referring to the disclosure herein and appropriately selecting a starting material, reagents, reaction conditions and the like as necessary.

If necessary, additives that are normally used for preparation of reagents may be blended to use the fluorescent probe as a composition. For example, as additives to be used in a physiological environment, additives such as a solubilizing agent, a pH adjusting agent, a buffering agent and an isotonizing agent can be used, and the blended amounts thereof can be appropriately selected by a person skilled in the art. These compositions can be provided as compositions in an appropriate form such as a powdered mixture, a freeze-dried product, a granule, a tablet or a solution.

### 3. Light emission mechanism of fluorescent probe molecule

Hereinafter, a fluorescence emission mechanism in the fluorescent probe of the present invention will be described.

The compound of the formula (I) has a skeleton of a xanthene-based fluorescent dye which is widely used for bioimaging because of its high water solubility, high fluorescence quantum yield and the like, but when the upper part of the xanthene skeleton is in a ring closure state, the fluorescent probe itself is substantially nonabsorbable and non-fluorescent (in a state in which the fluorescent response is off) in a neutral region (e.g. a pH range of 5 to 9). On the other hand, as shown in the following scheme, when a dipeptide site in P2-P1-NH is hydrolyzed by a protease and cut from a xanthene skeleton, a ring-opened tautomer is rapidly formed to produce a highly fluorescent compound (II).

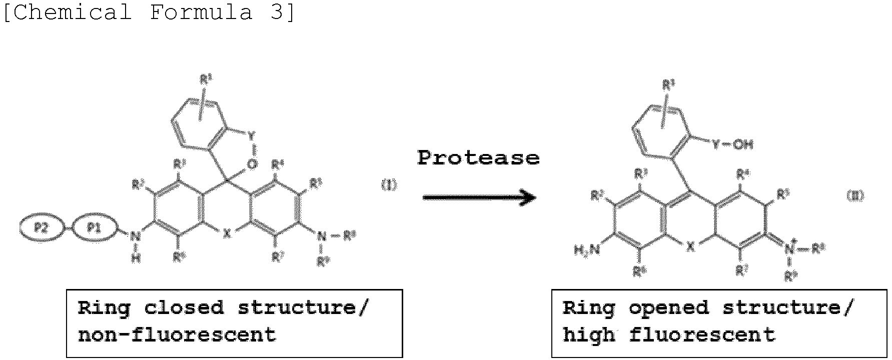

That is, the fluorescent probe of the present invention which contains the compound of the general formula (I) or a salt thereof as an active ingredient has a property of being hydrolyzed by a protease expressed in a brain tumor tissue to give the ring-opened compound (II) that emits strong fluorescence. Therefore, the presence of a brain tumor expressing the protease can be detected or labeled by using the fluorescent probe of the present invention. Examples of the protease include calpain 1 and cathepsin D as described previously.

More specifically, for example, the compound of the general formula (I) in which X is an O atom, or a salt thereof emits little fluorescence when irradiated with excitation light with a wavelength of, for example, about 440 to 510 nm in a neutral region, and the ring-opened compound has a property of emitting extremely strong fluorescence (e.g. emission: 524 nm) under the same condition. Therefore, when detection is performed using the fluorescent probe of the present invention, visible light with a wavelength of about 440 to 510 nm may be normally applied. The fluorescence wavelength to be observed is normally about 510 to 800 nm, and it is preferable to observe fluorescence of with a wavelength of, for example, about 516 to 556 nm.

### 4. Method for detection and visualization using fluorescent probe.

In accordance with such a light emission mechanism, a brain tumor expressing a specific protease can also be specifically detected or visualized using the fluorescent probe.

Specifically, the method of the present invention includes the steps of:
A) bringing the fluorescent probe into contact with a target tissue; and
B) observing a fluorescent response caused by a reaction between a protease expressed in the target tissue and the fluorescent probe. Only a target tissue (or target cell) expressing a specific protease can be specifically detected or visualized as a fluorescence signal. Herein, the term "detection" should be interpreted in the broadest sense including measurement for various purposes such as quantitative and qualitative determination.

As described previously, the particular protease may preferably be calpain 1 or cathepsin D. However, the protease is not limited thereto. The target tissue is a brain tumor tissue. Examples of the brain tumor include glioblastoma and glioma. Tumor cells can also be targeted.

In addition, the method of the present invention may include observing the fluorescent response using fluorescence imaging means. As the means for observing the fluorescent response, a fluorometer with a wide range of wavelengths measurement can be used, and the fluorescent response can also be visualized as a two-dimensional image by using fluorescence imaging . As for that matter, a target tissue (or cell) expressing the protease can be visually recognized instantly. As a fluorescence imaging apparatus, an apparatus known in the art can be used. In some cases, it is also possible to detect a reaction between the protease and the fluorescent probe by a change in ultraviolet-visible light absorption spectrum (e.g. a change in absorbance at a specific absorption wavelength).

The means for bringing a sample contact with the fluorescent probe is typically by spraying or addition or application of a solution containing the fluorescent probe to the sample. It can be appropriately selected according to the form of the sample, the measurement condition and the like. When a solution containing a fluorescent probe is sprayed to a sample, it is possible to use, for example, an apparatus with a storage unit that stores a chemical solution containing a fluorescent probe. Such an apparatus may be an endoscope. Examples of the endoscope having the aforementioned function include those as disclosed in Japanese Patent Laid-Open No. 2010-240188 and Japanese Patent Laid-Open No. 2015-23904. In addition, the apparatus may include a fluorescence imaging means as described previously for observing a fluorescent response by the fluorescent probe.

The applied concentration of the fluorescent probe of the present invention is not particularly limited, and for example, a solution with a concentration of about 0.1 to 10 µM can be applied.

As the fluorescent probe of the present invention, the compound of the aforementioned formula (I) or a salt thereof may be used as it is, and if necessary, additives that are normally used for preparation of reagents may be blended to use the fluorescent probe as a composition. For example, as additives for use of reagents in a physiological environment, additives such as a solubilizing agent, a pH adjusting agent, a buffering agent and an isotonizing agent can be used, and the blended amounts thereof can be appropriately selected by a person skilled in the art. These compositions can be generally provided as compositions in an appropriate form such as a powdered mixture, a freeze-dried product, a granule, a tablet or a solution, and may be dissolved in distilled water for injection or an appropriate buffer solution at the time of use.

The detection or visualization of a brain tumor by the method of the present invention can generally be performed under a neutral condition, for example in the pH range of 5.0 to 9.0, preferably in the pH range of 6.0 to 8.0, more preferably in the pH range of 6.8 to 7.6. As means for adjusting the pH, for example, any pH adjusting agent or buffer solution well known in the art such as a phosphate buffer can be used.

The method of the present invention can be used for the diagnosis of brain tumors by detecting or visualizing these tumors. The term "diagnosis" herein needs to be interpreted in the broadest sense including visual or microscopic confirmation of the presence of tumor tissue at any biological site.

The detection method of the present invention can be used, for example, during surgery or during examination. The term "surgery" herein includes any surgery applied for diagnosis and treatment of tumors, including, for example, craniotomy surgery with opening, burr hole opening surgery, open chest surgery, open abdominal, skin surgery, and surgeries under endoscopes such as neuroendoscopes, gastroscopes, colonoscopes, abdominal cavity scopes and thoracoscopes. In addition, the term "examination" includes examination using an endoscope and examinationrelated treatment such as excision and collection of tissues, examination performed outside a living body on tissues separated and collected from the living body, and the like.

As one of preferred aspects, for example, the fluorescent probe of the present invention is applied to a part or the whole of the field of surgery by spraying, application or injection under visual or endoscopic observation. The application site can be irradiated with light with a wavelength of about 500 nm after several tens of seconds to several minutes. When the application site includes a tumor tissue, the tissue will be identified as a tumor by emitting fluorescence, and therefore the tumor tissue will be excised with the surrounding area. For example, in surgical treatment of a tumor, definite diagnosis is performed on a tumor tissue that can be visually observed, and invasion or metastasis to a lymph node and surrounding organs or tissues can be diagnosed. In that way, it is possible to determine an excision area by performing intraoperative rapid diagnostic procedure.

As another preferred aspect, for example, the fluorescent probe of the present invention is applied to an examination part by an appropriate method such as spraying, application, or injection in endoscopic examination, and the application site is irradiated with light with a wavelength of about 500 nm for a few tens of seconds to several minutes. When a tissue emitting fluorescence is observed, the tissue can be identified as tumor. When tumor tissue can be observed in the endoscopic examination, the tissue can be subjected to excision for examination or therapeutic excision.

### [Examples]

Hereinafter, the present invention will be described in more detail by way of Examples, which should not be construed as limiting the present invention.

### [Example 1]

### 1. Synthesis of Fluorescent Probe

Hydroxymethyl rhodamine green (HRMG) having various dipeptide sites was synthesized in accordance with the reaction scheme described in Example 1 of WO 2016/006678. Compounds having different dipeptide sites were obtained by variously changing the Fmoc-amino acid used in the synthesis of compound A7 in Example 1.

The synthesized compound is one in which in the aformentioned formula (I), all of R¹, R², R³, R⁴, R⁵, R⁶, R⁷, R⁸ and R⁹ are hydrogen atoms, X is O, Y is a methylene group, and a combination of P1 and P2 is as follows.

**[Table 1]**

| **Compound** | **P1** | **P2** |
|---|---|---|
| **YK190** | **Arginine** | **Proline** |
| **YK213** | **Histidine** | **Glycine** |
| **YK19** | **Tyrosine** | **Glycine** |
| **YK281** | **Methionine sulfoxide** | **Acetylleucine** |

### [Example 2]

### 2. Screening by fluorescent probe

The fluorescent intensity of fresh glioblastoma and peritumoral tissue were compared using the fluorescent probes YK190, YK213, YK19, and YK281 synthesized in Example 1. As a comparative example, hydroxymethyl rhodamine green (HRMG) having no dipeptide site was used.

50 µM probe solutions (200 µL) in PBS, containing 0.5% v/v DMSO as a co-solvent was added dropwise to each specimen, and the fluorescence intensity was measured over time using Maestro In Vivo Imaging System Ex.
- Excitation wavelength: 465 nm
- Emission wavelength: 515 nm

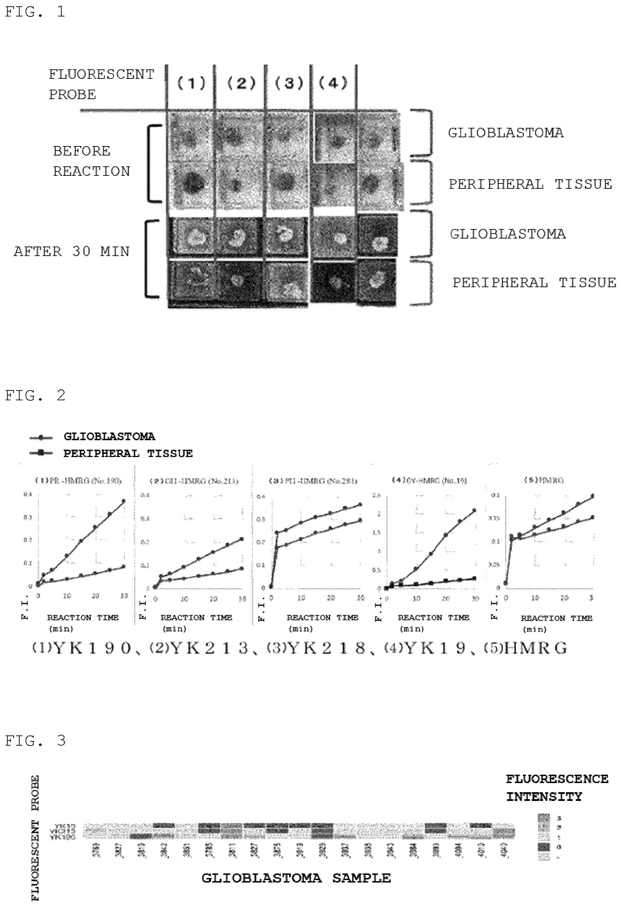

As a result, as shown in Figs. 1 and 2, fluorescent labeling was performed in a tumor-specific manner as compared with the surrounding tissue for (1) YK190 and (2) YK213, whereas there was no significant difference as compared with the surrounding tissue for (3) YK218. (5) HRMG is a comparative example having no dipeptide site on the side chain. As shown in Fig. 2, both YK190 and YK213 exhibited a high fluorescence intensity in the tumor, and YK190 was found to be a more preferred fluorescent probe because it exhibited a fluorescence intensity about two times that of YK213. For (4) YK19, a fluorescent response was also well-obtained in a tumor-specific manner.

Fig. 3 collectively shows the ratio of fluorescent reactions with tumor and peritumoral tissue using synthesized probes.(the abscissa represents a sample, the ordinate represents a probe name, and samples for which no fluorescent response was described in zero).

### [Example 3]

### 3. Identification of protease

Next, the protease involved in the fluorescent response obtained in Example 2 was identified.

Protease analysis was performed by a DEG (diced electrophoresis gel) assay and peptide mass fingerprinting using LC MS/MS (liquid chromatograph - mass spectrometry) and as candidate enzymes, cathepsin D, cytosolic nonspecific dipeptidase, calpain 1 and cytosolic aminopeptidase were identified.

For these candidate enzymes, a change in fluorescence intensity was detected using inhibitors (SNJ-1945 and Amastatin), and the results showed that fluorescence suppression by SNJ-1945 occurred for calpain 1 and cathepsin D.

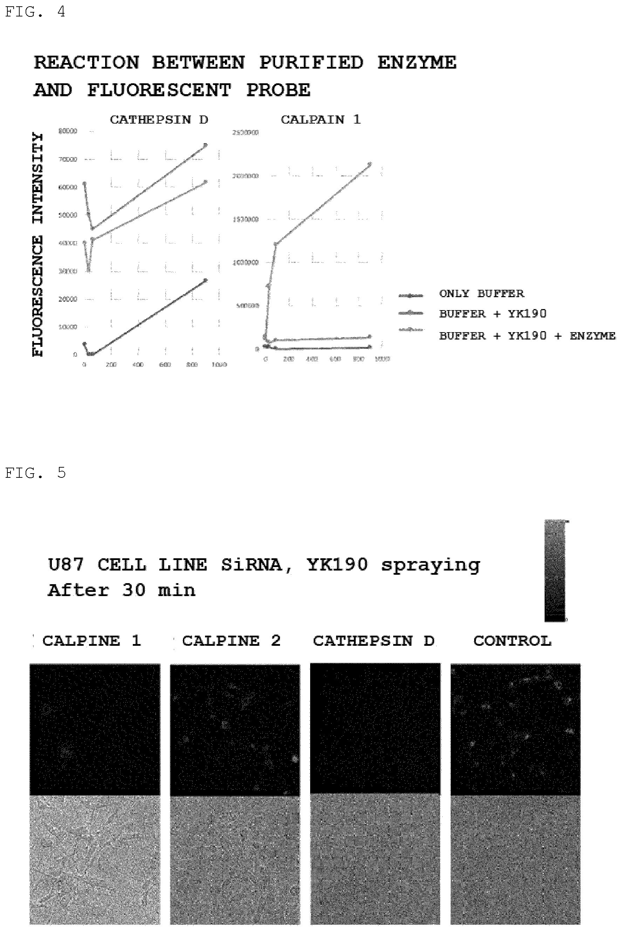

For calpain 1 and cathepsin D purified enzymes, a change in fluorescence intensity by the reaction with the fluorescent probe YK190 was measured, and the results showed that, as shown in Fig. 4, a significant increase in fluorescence intensity was observed. Therefore, it was suggested that the fluorescent response to glioblastoma in Example 2 arose from the reaction between these enzymes and the fluorescent probe.

In addition, in searching for expression enzymes by real-time PCR, an increase in gene expression of calpain 1 and cathepsin D was observed in the tumor lysate as compared with the peripheral tissue lysate. These results were consistent with the previously-described results.

### [Example 4]

### 4. Verification by SiRNA using U 87 cell line

Calpain 1 and cathepsin D were knocked down by siRNA in a cell line, and a reaction with the fluorescent probe YK190 was observed. The experiment was conducted in accordance with the procedure shown below.

Transfection of U87, a glioblastoma cell line, was done with siRNA utilizing lipofectamine. A reaction with a fluorescent probe was observed after culture for 48 hours.

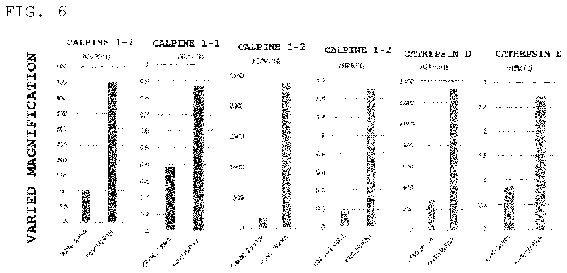

As a result, the cell line in which either calpain 1 or cathepsin D was knocked down by siRNA showed reduced fluorescence intensity when incubated with YK190 compared to the control U87 cell line (Fig. 5). From these results, it was suggested that the fluorescent response to glioblastoma in Example 2 arose from the reaction between these enzymes and the fluorescent probe. Further, as an experiment for supporting the aforementioned results, the enzyme expression level was examined by real-time PCR, and a consistent result was obtained (Fig. 6).

## Claims

1. A fluorescent probe for detecting a brain tumor, comprising of a compound of the following formula (I) or a salt thereof: 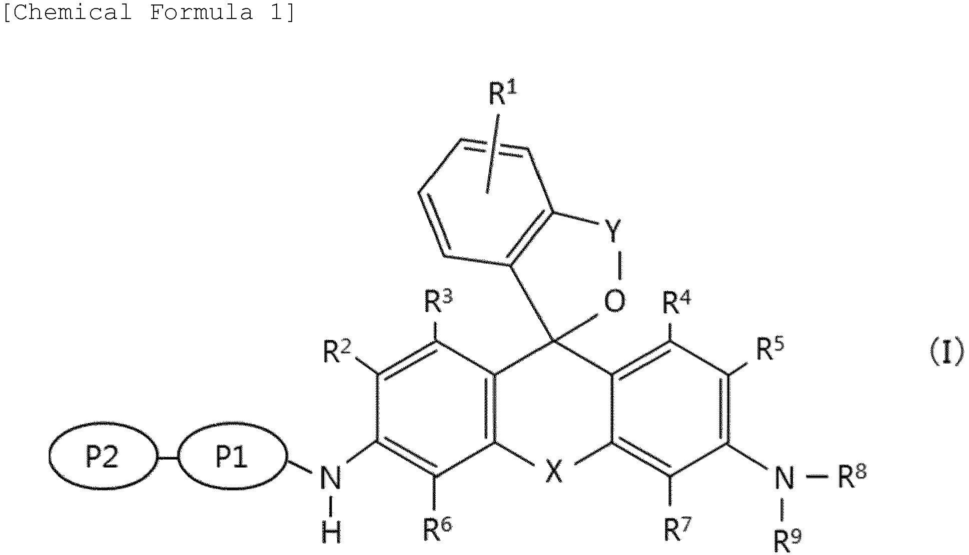
wherein P1 represents an arginine residue, a histidine residue or a tyrosine residue, P2 represents a proline residue or a glycine residue, where P1 is linked to an adjacent N atom by forming an amide bond, and P2 is linked to P1 by forming an amide bond;
R¹ represents a hydrogen atom, or 1 to 4 identical or different substituents each independently selected from the group consisting of an optionally substituted alkyl group, a carboxyl group, an ester group, an alkoxy group, an amide group and an azide group;
R², R³, R⁴, R⁵, R⁶ and R⁷ each independently represent a hydrogen atom, a hydroxyl group, an optionally substituted alkyl group or a halogen atom; R⁸ and R⁹ each independently represent a hydrogen atom or an alkyl group;
X represents O, Si (R^{a})(R^{b}), Ge (R^{a})(R^{b}), Sn(R^{a})(R^{b}), C(R^{a})(R^{b})or P(=O)(R^{a});
R^{a} and R^{b} each independently represent a hydrogen atom, an alkyl group or an aryl group; and
Y represents a C₁-C₃ alkylene group.

2. The fluorescent probe according to claim 1, wherein the brain tumor is glioblastoma or glioma.

3. The fluorescent probe according to claim 1 or 2, for identifying a brain tumor or glioma by detecting the presence of a protease expressed in tumor cells.

4. The fluorescent probe according to claim 3, wherein the protease is calpain 1 or cathepsin D.

5. The fluorescent probe according to any one of claims 1 to 4, wherein X is O (oxygen atom) and Y is a methylene group.

6. The fluorescent probe according to any one of claims 1 to 5, wherein all of R¹, R², R³, R⁴, R⁵, R⁶, R⁷, R⁸ and R⁹ are hydrogen atoms.

7. An application-type or topical spray-type composition for detection of a tumor, including a solution of the fluorescent probe according to any one of claims 1 to 6.

8. A method for detecting or visualizing a brain tumor, comprising the steps of:
bringing the fluorescent probe according to any one of claims 1 to 6 into contact with a target tissue; and observing a fluorescent response caused by a reaction between a protease expressed in the target tissue and the fluorescent probe.

9. The method according to claim 8, wherein the step of bringing the fluorescent probe into contact with the target tissue is carried out by topically applying or spraying a solution containing the fluorescent probe to the target tissue.

10. The method according to claim 8 or 9, comprising visualizing the fluorescent response using fluorescence imaging means.

11. The method according to any one of claims 8 to 10, wherein the brain tumor is glioblastoma or glioma.

12. The method according to any one of claims 8 to 11, wherein the protease expressed in the target tissue is calpain 1 or cathepsin D.
